# EUROPEAN PATENT APPLICATION

(11) **EP 4 300 503 A1**
(43) Date of publication of application: **03.01.2024**
(21) Application number: 22181302.5
(22) Date of filing: 27.06.2022
(51) Int. Cl.: G16C 60/00

(54) **SYSTEM AND METHOD FOR PROCESSING MATERIAL PROPERTIES OF STRUCTURAL MATERIALS**

(71) Applicant: Total Materia AG, 8052 Zürich (CH)
(72) Inventor: Pocajt, Viktor, 8052 Zürich (CH); Antanasijevic, Davor, 8052 Zürich (CH)
(74) Representative: Rentsch Partner AG

(57) **Abstract**

A computer implemented method for processing material properties of structural materials, the method comprising: generating training dataset(s) for training a machine learning algorithm for determining values(s) of specific target material property(s) of an assessment material; training a machine learning algorithm for determining value(s) of specific target material property(s) of an assessment material using the training dataset; and determining value(s) of specific target material property(s) of an assessment material using the trained machine learning model.

## Description

### TECHNICAL FIELD

The present disclosure relates to a method, a system and a computer program product for processing material properties of structural materials. Specifically, the present disclosure relates to a method, a system and a computer program product for processing material properties comprising: generating training dataset(s) for training a machine learning algorithm; training the machine learning algorithm with the training dataset for determining material properties of a structural material using the training dataset(s); and determining material properties of a structural material using the machine learning model obtained by training of the machine learning algorithm.

### TECHNICAL BACKGROUND

Properties of structural materials are of vital importance for the design and use of diversified products such as machinery, structures, vehicles, aerospace products and many others. Macroscopic mechanical properties such as yield and tensile strength, toughness and hardness, as well as physical properties such as density, modulus of elasticity and electrical and thermal conductivity are necessary to engineers to determine suitability of a material for a certain application, to calculate dimensions of structures, parts and elements and to simulate their behavior in exploitation.

In many cases, some or all of these properties for materials of interest are not available, which may seriously impede engineering analysis, calculations and simulations. Whilst mechanical, physical and other properties can be obtained experimentally, testing is in many cases too costly and too time consuming. Therefore, there is a need to predict properties as an alternative and/or supplement to material testing.

There is a great number of different mathematical models for some particular mechanical and physical properties, for some specific materials and frequently for some conditions. Their applicability and accuracy are usually limited and, more recently, methods based on artificial intelligence and machine learning have been applied in this field in order to improve properties modeling and prediction.

### SUMMARY OF THE DISCLOSURE

It is an object of this disclosure to provide a computer implemented method, a system and/or a computer program product for processing material properties of structural materials. In particular, it is an object of the present disclosure to provide a method for processing material properties of structural materials, which method does not have at least some of the disadvantages of the prior art.

According to the present disclosure, these objects are addressed by the features of the independent claims. In addition, further advantageous embodiments follow from the dependent claims and the description.

It is an object of this disclosure to provide a computer implemented method, a system and/or a computer program product for generating training dataset(s) for training a machine learning algorithm for determining value(s) of specific target material property(s) of an assessment material.

These objects are addressed by a computer implemented method, a system and/or a computer program product of generating training dataset(s) for training a machine learning algorithm for determining value(s) of specific target material property(s) of an assessment material, in particular as part of a method, a system and/or a computer program product for processing material properties of structural materials. The generation of training dataset(s) comprises providing a database comprising data records indicative of values of material properties of a multitude of structural materials. The structural materials can be steel, aluminum, other nonferrous metals,polymers and other non-metallic materials such as ceramics as well as combinations and compositions of these materials. The material properties can be any parameter describing the material. The material properties can be intrinsic properties as any physical, mechanical, electromagnetic, optical and/or other properties of structural materials. The material properties might also be extrinsic material properties such as material conditions that can include how the material was produced and/or processed (e.g. heat treatment, shape, thickness such as a combination of thermic-, mechanical- and other processing patterns), how one or more of the material properties has/have been measured, how it is used (e.g. temperature range, pressure, humidity) and environmental conditions of use (e.g. corrosive environment, axis of orientation). Another type of material property might also be a material indicator as a material type as defined by a composition (e.g. steel, aluminum etc.), to a higher or lower granularity (e.g. low carbon steel high alloyed, low carbon steel low alloyed etc.), and/or by a pre-defined classification (e.g. classification into metals, polymers, ceramics, and composites).

The training dataset(s) is/are dedicated for one instance of target properties, there is not a single training dataset for training a machine learning algorithm to determine any target property. In other words, the training dataset(s) are generated according to the present invention for a particular purpose, i.e. for training a machine learning algorithm to determine the specific target material property(s).

The generation of training dataset(s) further comprises classifying the data records into a plurality of material classes according to the value(s) of one or more of the material properties, the value(s) being identical or similar. For example, identical values of the property "material type" might be a material property to use for classifying materials into material classes. Similarity can for example be determined by the value(s) being within a range according to a distance metric. In particular, the material type might be a material property to classify materials into material classes.

According to embodiments, the data records are classified (iteratively) into a plurality of material classes according to values of a plurality of the material properties using a hierarchical taxonomy. For example, according to material groups, such as Level 1 in the case of nonferrous and Level 2 and/or Level 3 in the case of ferrous metals, or material family Level 2, 3 and 4 for polymers and other nonmetals.

The generation of training dataset(s) further comprises extracting target-relevant material properties within one or more of the material classes. The target-relevant material properties are extracted from the data records corresponding to the specific target material property(s). In the context of the present application, target-relevant material properties are a subset of all material properties in the data records within a material class. A material property in the data records within a material class is part of the subset referred to as target-relevant material properties, if it has any dependency relationship with the specific target material property(s). Hence, the same database, the same material class may be result in a different set of target-relevant material properties for different target material properties. For example, the material property "color" would not be part of the subset of target-relevant material properties - within the material class of metals - for the specific target material property of e.g "tensile strength". However, the same material property "color" would very well be part of the subset of target-relevant material properties - within the material class of metals - for the specific target material property of e.g. "light absorption".

The generation of training dataset(s) also comprises determining the training dataset(s) corresponding to one or more of the material classes using values of the extracted target-relevant material properties and corresponding value(s) of the specific target material property(s). In other words, the training dataset(s) shall comprise the values of specific target material property(s) together with their respective target-relevant material property(s), i.e. the material property(s) with a dependency relationship with the target material property(s). Material properties with no dependency relationship with the target material property(s) are discarded and only a subset of the property-relevant material properties of the data records having a dependency relationship with the target material property(s) is added to the training dataset(s).

The method of processing material properties according to the present invention is particularly advantageous due to its broad applicability, i.e. its ability to cover broad categories of materials. Depending on the amount of available data records of the database (up to hundreds of thousands of different materials) with a suitably complex hierarchical taxonomy for classification, machine learning algorithms can be trained for determining potentially dozen(s) of target material property(s) of a very large number of materials.

In particular, determining training dataset(s) as a substep of generating training dataset(s) may comprise transfer of the target-relevant material properties of data records indicative of values of material properties, or the data records can be subject to normalization before inserted into the training dataset(s). For implementations of normalizing data, see the embodiment below.

In an embodiment, the generation of training dataset(s) further comprises determining validation and/or test dataset(s) corresponding to one or more of the material classes using values of the extracted target-relevant material properties and corresponding value(s) of the specific target material property(s). In particular, as with the determination of training dataset(s), the determination of validation dataset(s) and test dataset(s) may comprise transfer of the data records indicative of values of material properties, or the data records can be subject to normalization before inserted into the training dataset(s). For implementations of normalizing data, see the embodiment below.

In an embodiment, as part of the generation of training dataset(s), the splitting of one or more of the material classes into training, validation and/or test dataset(s) is performed using a rational split algorithm, such as selection of data records within one or more of the material classes having a uniform distribution of values of one or more of the material properties over a regression space. In particular, a Kennard-Stone algorithm or other rational split algorithms can be used to produce a training subset, while the remaining data are further randomly split into validation and test datasets.

In an embodiment, as part of the generation of training dataset(s), the extraction of target-relevant material properties from the data records indicative of material properties of one or more of the material classes comprises applying a mutual information based algorithm, in particular a partial mutual information algorithm to identify a subset of target-relevant material properties of the data records having a dependency relationship with the target material property(s).

Alternatively, or additionally, as part of the generation of training dataset(s), the extraction of target-relevant material properties from the data records indicative of material properties of one or more of the material classes comprises applying a selection algorithm, such as filter, embedded or wrapper-type selection algorithms onto the data records within one or more of the material classes to identify a subset of target-relevant material properties (out of all material property(s)) of the data records having a dependency relationship with the target material property(s). For example, applying a Markov blanket algorithm onto the data records within one or more of the material classes comprises identifying a Markov blanket, i.e. the subset of material properties that comprises all data useful for determining the specific target material property(s). Alternatively, or additionally, beam search, Sequential Forward Selection, and/or a genetic algorithm is applied to onto the data records within one or more of the material classes to identify a subset of target-relevant material properties (out of all material property(s)) of the data records having a dependency relationship with the target material property(s).

In an embodiment, the generation of training dataset(s) further comprises normalizing the data records indicative of material properties within one or more of the material classes, wherein normalizing the data records within one or more of the material classes comprises at least one of: removal of data records with missing values of material properties, removal of data records comprising extreme values of material properties, and/or removal of data records comprising inconsistent values of material properties. In particular, extreme values are outliers and differ significantly from the majority of other data. Inconsistent values can occur occasionally for some physical and mechanical properties that have substantially different values for same or similar conditions. This can be a consequence of experimental errors, errors in data and unit conversions and the like.

In an embodiment, normalizing the data records within one or more of the material classes further comprises transforming non-numerical values of material properties into corresponding numerical representations. In particular, the step of transforming non-numerical values of material properties into corresponding numerical representations is performed by applying a suitable encoding, such as a binary, numeric or target based encoding. This step may also involve augmentations and high cardinality categorical variables transformation to its numerical representation. Such a transformation of non-numerical values into corresponding numerical values advantageously is applied in a consistent manner across materials and properties in order to be comparable. The transformation also should be used consistently over time, as a change could lead to the whole data processing having to be repeated again.

According to embodiments disclosed herein, the method of processing material properties of structural materials comprises determining respective applicability domain(s) of one or more of the training dataset(s). Defining the applicability domain corresponding to a training dataset is important in order to determine the material properties that could be reliably predicted using a machine learning algorithm trained with the respective training dataset. The applicability domain corresponding to a training dataset defines/ delimits or even one to one corresponds to the applicability domain of a machine learning algorithm trained with the respective training dataset. The degree of generalization of the machine learning algorithm depends on how broad its domain of applicability is. If the applicability domain is too restricted, this implies the machine learning algorithm is capable of providing reliable predictions only for limited number of cases, which embodiments of the present invention intend to overcome. Therefore, the characterization of interpolation space is significant in defining the applicability domain.

In an embodiment, the applicability domain of a training dataset is determined by applying a range-based approach. According to the range-based approach, respective ranges of the values of target-relevant material properties within a training dataset are used to define boundaries of the applicability domain of a training dataset. Assuming a uniform distribution, the applicability domain is defined by a p-dimensional hyper-rectangle (p being the number of property-relevant features within a training dataset corresponding to the specific target material property(s)), the edges of the p-dimensional hyper-rectangle being defined on the basis of maximum and minimum values of each material property. The sides of this hyper-rectangle are parallel with respect to coordinate axes corresponding to each material property within the training dataset. The hyperspace within the p-dimensional hyper-rectangle delimits the applicability domain of the respective training dataset for training machine learning algorithm to predict target material property(s) of a structural material.

It is a further object of this disclosure to provide a computer implemented method, a system and/or a computer program product for training a machine learning algorithm, for processing material properties of structural materials.

This further object is addressed by a computer implemented method, system and/or computer program product of training of a machine learning algorithm for determining the specific target material property(s) of an assessment material using the training dataset(s), in particular as part of the computer implemented method, system and/or computer program product for processing material properties of structural materials.

In an embodiment, the training of the machine learning algorithm comprises computing a regression model representative of a relationship between values of two or more material properties within the training dataset(s). In particular, a first material property value is a later input value and another second material property value is a later output value of the trained machine learning model that was trained during the training of the machine learning algorithm. As an example, the first material property values that are input might be a material type and extrinsic material properties such as a temperature range and a processing procedure the material was subject to, and the second material property value might be the output value being the tensile strength of the material in question.

In an embodiment, the training of the machine learning algorithm comprises computing and compiling machine learning model(s) having a specific architecture. The machine learning model(s) is/are computed in the way that is specific for the specific architecture. After that, the model(s) is/are coded and compiled, referred to as a "trained" machine learning algorithm or a machine learning model.

In an embodiment, different machine learning models are combined applying assembled learning paradigm.

In an embodiment, based on the above embodiments, the training of the machine learning algorithm further comprises evaluating a machine learning model having a specific architecture. Thereby, architecture-specific approaches can be used to enhance the model performance with the adjustment of parameters. The performance of the obtained model is evaluated on the test subset, which comprises of dataset(s) that has / have not been introduced to the model in the training process. When the satisfactory performance is level is achieved, the model is coded and compiled, referred to as a "trained" machine learning model.

Architecture-specific parameters are initialized prior to running and optimizing the model and optimized during learning. For the example of the machine learning model being a neural network, examples of parameters are connection weights between layers, the number of layers and the number of nodes per layer in the neural network. During training, these parameters are updated and optimized using the training dataset(s), optimizing towards a minimal error determined by a predefined cost function.

In order to prevent overtraining of the machine learning algorithm, according to embodiments, the error is determined - using the cost function - also for the validation dataset(s), and the training process is halted as soon as an overtraining threshold is reached. The overtraining threshold is typically at a crossover point at which the error associated with the training dataset(s) is decreasing but the error associated with the validation dataset(s) is increasing. This halt is done to prevent overtraining.

In an embodiment, a neural network - comprised by the machine learning algorithm - comprises a plurality of successive layers comprising at least an input layer, a hidden layer, and an output layer. The neural network further comprises a plurality of weight matrices indicative of connection weights between successive pairs of layers. The training of the machine learning algorithm further comprises defining a cost function of the neural network. Having defined the cost function, an input is presented to the input layer of the neural network, the input having an associated expected output. The input comprises a first subset of material properties of any one of the data records of the training dataset(s) and the expected output comprises a second subset of material properties of the plurality of data records of the training dataset(s). Thereafter, a generated output is determined at the output layer by applying the machine learning algorithm to the presented input.

Having determined the generated output, value(s) of the cost function is/are determined based on a comparison of the expected output and the generated output.

Thereafter, the neural network is adapted based on the value(s) of the cost function, such adapting the weights and/ or biases of one or more pair(s) of successive layers and/or adapting the number of layers of the neural network.

In an embodiment, as part of the training of the machine learning algorithm comprising a neural network, the steps of the machine learning algorithm described in the above embodiment are run on the neural network iteratively for a plurality of data records of the training dataset(s).

In an embodiment, the neural network is a General Regression Neural Network and adapting one or more pair(s) of successive layers of the neural network comprises optimizing the weights of the one or more pair(s) of successive layers of the neural network using genetic algorithm(s).

In an embodiment, the neural network is a Deep Neural Network, and adapting one or more pair(s) of successive layers of the neural network comprises selection of meta parameters being a type of parameters using meta parameter optimization library(s). For example, if the Deep Neural Network is TabNet, iterative algorithms can be used to find optimized meta parameters. Alternatively or in combination, specialized libraries such as Optuna can be used to select the meta parameters.

In an embodiment, the training of the machine learning algorithm further comprises initializing the neural network with random connection weight values. In particular, the neural network is initialized using random values from a uniform distribution over [-a, a] where a is a scalar value.

In an embodiment, the machine learning algorithm comprises a decision tree-based machine learning algorithm, in particular a gradient-boosted decision tree-based machine learning algorithm. An example of such an embodiment is the use of Jboost, a boosting algorithm for Java implementations, or XGBoost, a distributed boosting algorithm.

In an embodiment, the training of the machine learning algorithm further comprises determining a halt of the training process using the validation dataset(s) and/or evaluating the performance of the machine learning algorithm using the test dataset(s).

According to an embodiment, in case of a neural network-based machine learning algorithm, the steps of presenting an input (comprising data records of the training dataset(s)) to the input layer, determining a generated output, determining value(s) of the cost function, adapting the neural network and evaluating the performance of the neural network-based machine learning algorithm using the validation dataset(s) is performed repeatedly until a satisfactory performance is level is achieved. Thereafter, the neural network-based machine learning algorithm is coded and compiled, referred to as a "trained" neural network-based machine learning algorithm or neural network-based machine learning model.

It is an even further object of this disclosure to provide a computer implemented method, a system and/or a computer program product for determining value(s) of the specific target material property(s) of an assessment material.

This even further object is addressed by a computer implemented method, a system and/or a computer program product for determining value(s) of the specific target material property(s) of an assessment (structural) material using a machine learning algorithm, in particular as part of the computer implemented method, system and/or computer program product for processing material properties of structural materials.

In an embodiment, the determination of value(s) of the specific material property(s) of an assessment material comprises two steps. One step is receiving input indicative of value(s) of a first (known) set of material properties of the assessment material and another step is determining value(s) of the specific target material property(s) of the assessment material using the machine learning algorithm trained using a training dataset(s) determined corresponding to the specific target material property(s). Thereby, the machine learning algorithm was trained according to any of the above mentioned embodiments of training a machine learning algorithm using training dataset(s) generated according to any one of the embodiments of generating training dataset(s).

In an embodiment, building on the aforementioned embodiment, the determination of value(s) of specific target material property(s) using the machine learning algorithm further comprises selecting a machine learning algorithm trained with a training dataset(s) for the specific target material property(s).

In particular, a machine learning algorithm is selected which has been trained using training dataset(s) - and possibly validation dataset(s) and test dataset(s) - generated with respect to material class(s) classified according to a material property common with a known material property of the assessment material. For example, if the specific target material property is a condition property such as a temperature range, a corresponding assessment condition property comprises temperature range(s) equal to and/or comprised by and/or overlapping with temperature range. Thereafter, the value(s) of the specific target material property(s) of the assessment material is determined using the selected machine learning algorithm.

In particular, in one alternative, the selected machine learning algorithm (based on a machine learning model) out of the plurality of machine learning algorithms is that one that had achieved a highest score in the evaluation using the test dataset(s). In another alternative, the selected machine learning algorithm is the one that is based on combined machine learning models applying assembled learning paradigm.

In an embodiment, the determination of value(s) of target material property(s) using the machine learning algorithm further comprises receiving a selection, in particular a user selection, of a machine learning model or a combination of machine learning models. Therein, the machine learning algorithm is/was generated and trained in accordance with the selection of a machine learning model(s) and the values of specific target material property(s) of the assessment material is/were determined using the machine learning algorithm according to the selected machine learning model(s). The selection of a machine learning model amongst a plurality of machine learning models defines structure of the machine learning algorithm. The values of specific target material property(s) of the assessment material is/were then determined using the machine learning algorithm generated according to the selected machine learning model(s).

In an embodiment, the method for processing material properties of structural materials further comprises generating a data output indicative of the values of the target material property(s) of the assessment material.

In an embodiment, the method for processing material properties of structural materials comprises displaying a visual representation of at least part of the data output on a display device. Additionally, or alternatively the method for processing material properties of structural materials further comprises manufacture of a product comprising the target material property in accordance with the determined values of the target material property(s) of the assessment material.

In addition to the method for processing material properties of structural materials, it is a further object of the present disclosure to provide a system for processing material properties of structural materials. In particular, it is an object of the present disclosure to provide a system, which system does not have at least some of the disadvantages of the prior art. This further object is addressed by a system for processing material properties of structural materials comprising a data storage device for storing a database comprising data records indicative of values of material properties of a multitude of structural materials, and for storing training dataset(s). The system also comprises a computing device communicatively connected to the data storage device, an input interface for receiving input indicative of value(s) of a first set of material properties of the assessment material and/or for receiving a selection of a machine learning model. The system further comprises an output interface for outputting output data indicative of values of target material property(s) of an assessment material. The computing device comprises any one or a combination of: a central processing unit CPU, a physical or virtual computing device, a remote computing service, such as a cloud based software as a service, and/or a dedicated hardware circuitry, such as an ASIC or FPGA. The data storage device comprises any one or a combination of: a volatile or non-volatile, optic, magnetic or semiconductor based data storage device, and/or a cloud based datastore. The system is configured to carry out the method for processing material properties of structural materials according to any one of the embodiments described above.

In a further embodiment, the data storage device of the system for processing material properties of structural materials is further configured to store validation dataset(s) and/or testing dataset(s).

In an embodiment, the system for processing material properties of structural materials further comprises a display device communicatively coupled to the output interface for displaying a visual representation of the output data.

In addition to the method for processing material properties of structural materials and a system therefor, it is an even further object of the present disclosure to provide a computer program product comprising computer program code for processing material properties of structural materials. In particular, it is an object of the present disclosure to provide a computer program product, which computer program product does not have at least some of the disadvantages of the prior art. This even further object is addressed by a computer program product comprising instructions which, when executed by a computing device, cause the computing device to carry out the method of processing material properties of structural materials according to any one of the embodiments disclosed herein.

### BRIEF DESCRIPTION OF THE DRAWINGS

The present disclosure will be explained in more detail, by way of example, with reference to the drawings in which:
- Figure 1:: shows a block diagram illustrating schematically a system for processing material properties of structural materials;
- Figure 2:: shows a flow diagram illustrating an exemplary sequence of steps for processing material properties of structural materials;
- Figure 3a:: shows a flow diagram illustrating an exemplary sequence of steps for a method for generating material property(s) training dataset(s);
- Figure 3b:: shows another flow diagram illustrating an exemplary sequence of steps for a method for generating material property(s) training dataset(s);
- Figure 3c:: shows another flow diagram illustrating an exemplary sequence of steps for a method for generating material property(s) training dataset(s);
- Figure 3d:: shows another flow diagram illustrating an exemplary sequence of steps for a method for generating material property(s) training dataset(s);
- Figure 4a:: shows a schematic block diagram illustrating an exemplary generic machine learning model architecture for an exemplary machine learning algorithm that is based on such a machine learning model architecture;
- Figure 4b:: shows a schematic block diagram illustrating an exemplary neural network for an exemplary machine learning algorithm that is based on a neural network;
- Figure 4c:: shows a flow diagram illustrating an exemplary sequence of steps for a method of training a machine learning algorithm for determining material property(s) of an assessment material using training dataset(s);
- Figure 4d:: shows another flow diagram illustrating an exemplary sequence of steps for a method of training a machine learning algorithm for determining material property(s) of an assessment material using training dataset(s);
- Figure 4e:: shows another flow diagram illustrating an exemplary sequence of steps for a method of training a machine learning algorithm for determining material property(s) of an assessment material using training dataset(s);
- Figure 4f:: shows another flow diagram illustrating an exemplary sequence of steps for a method of training a machine learning algorithm for determining material property(s) of an assessment material using training dataset(s);
- Figure 5a:: shows a flow diagram illustrating an exemplary sequence of steps for a method for determining material properties of an assessment material using a machine learning algorithm; and
- Figure 5b:: shows another flow diagram illustrating an exemplary sequence of steps for a method for determining material properties of an assessment material using a machine learning algorithm.

### DETAILED DESCRIPTION OF EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that his disclosure will satisfy applicable legal requirements. Whenever possible, like reference numbers will be used to refer to like components or parts.

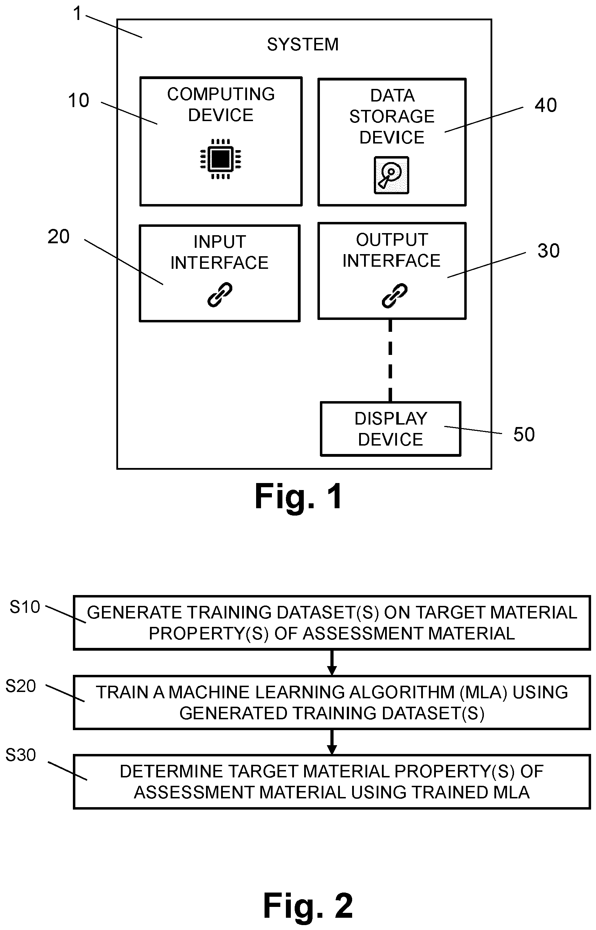

**Figure 1** shows a block diagram illustrating schematically a system 1 for processing material properties of structural materials. The system 1 comprises at least one computing device 10, a data storage device 40, an input interface 20 and an output interface 30. The system 1 optionally comprises a display device 50.

The data storage device 40 comprises instructions, which, when executed by the computing device 10, cause the computing device 10 to carry out the method according to one of the embodiments disclosed herein. The computing device 10 comprises any one or a combination of: a central processing unit CPU, a physical or virtual computing device, a remote computing service, such as a cloud based software as a service, and/or a dedicated hardware circuitry, such as an ASIC or FPGA. The data storage device 40 comprises any one or a combination of: a volatile or non-volatile, optic, magnetic or semiconductor based data storage device, and/or a cloud based datastore.

The input interface 20 is communicatively connected to the computing device 10 and the data storage device 40. The input interface 20 is any one of or a combination of a wired (e.g. Ethernet, USB, DVI, HDMI, VGA) and/or wireless data communication interface (e.g. 4G, 5G, Wifi, Bluetooth, UWB). The input interface 20 is used to provide input data in form of files or user selections to the computing device 10 and the data storage device 40.

The system 1 further optionally comprises a display device 50, such as a computer screen, or any device suitable to display a visual representation of (at least part of) data output indicative of material properties of an assessment material, the display device 50 being communicatively connected to the computing device 10 via an output interface 30. The output interface 30 is any one of or a combination of a wired (e.g. Ethernet, USB, DVI, HDMI, VGA) and/or wireless data communication interface (e.g. 4G, 5G, Wifi, Bluetooth, UWB) and is configured to transmit at least part of the assessment, respectively a visual representation of the assessment.

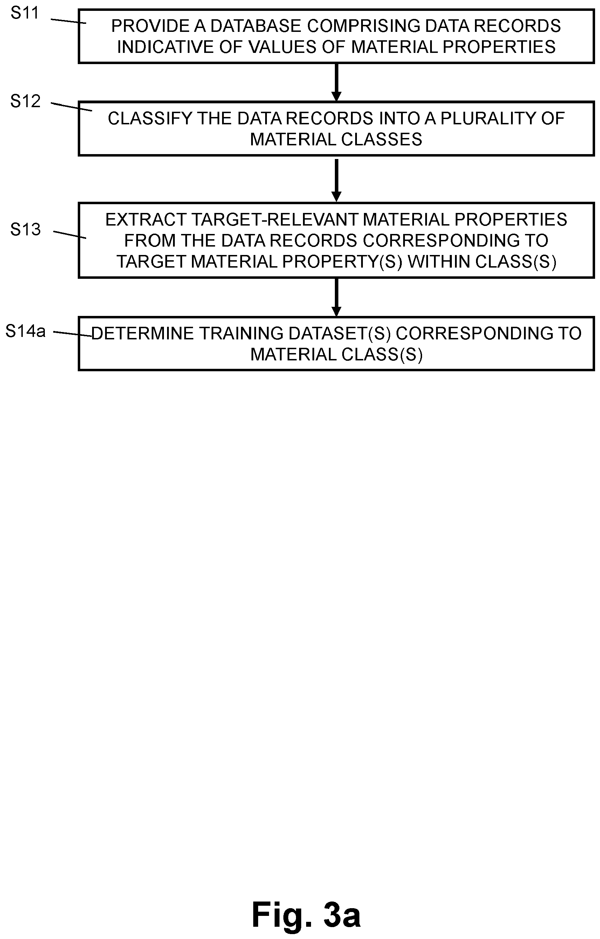

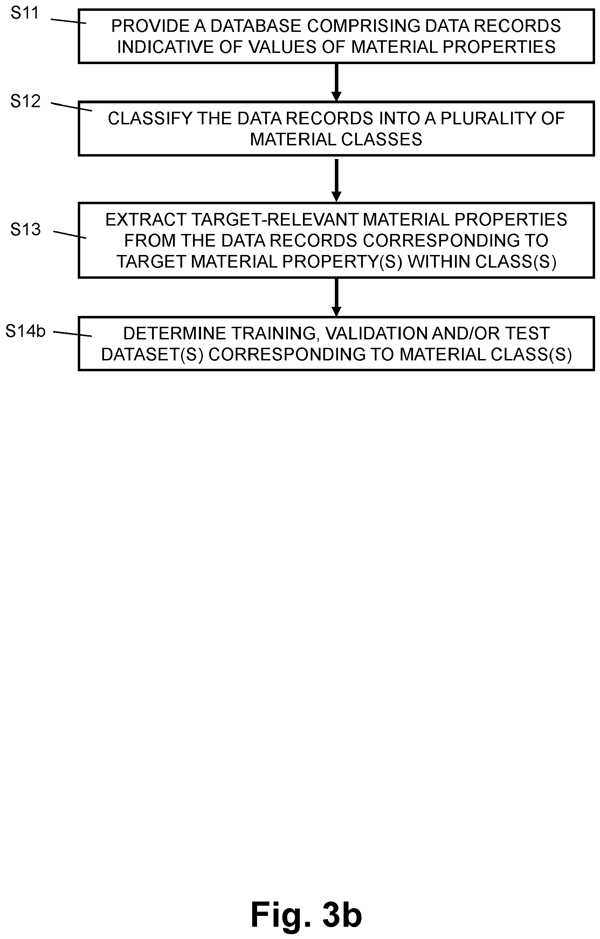

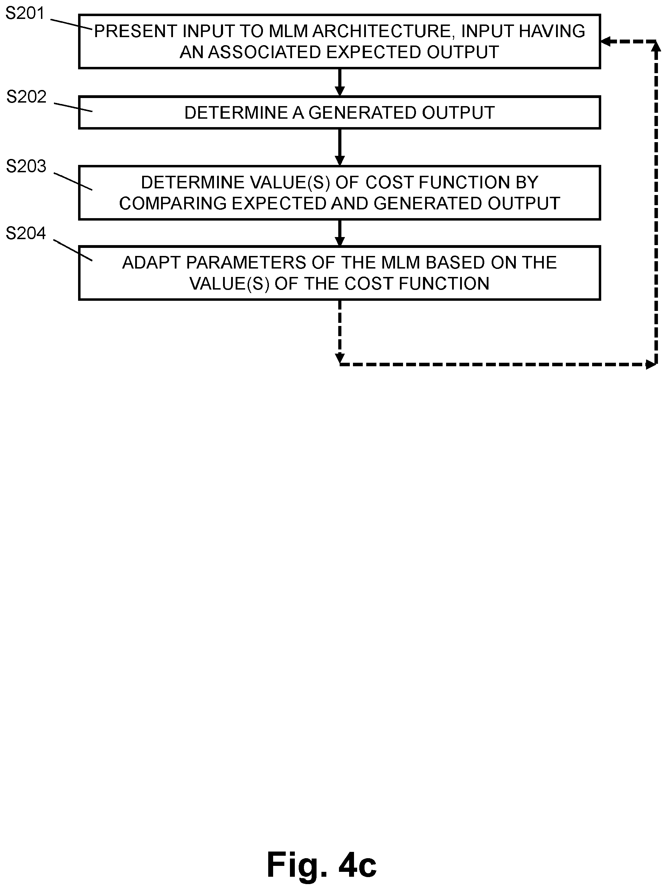

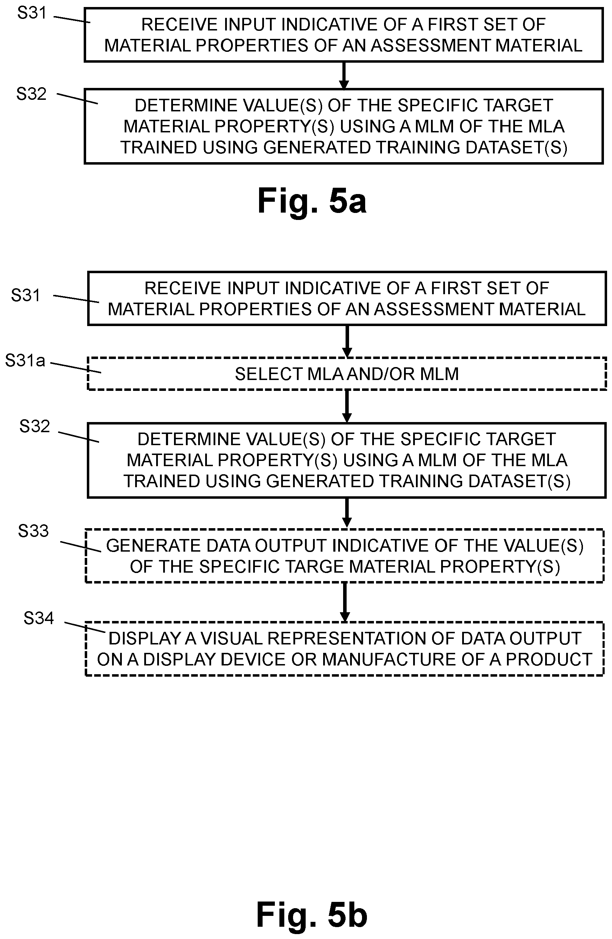

**Figure 2** shows a flow diagram illustrating exemplary sequence of steps for processing material properties of structural materials. In a first step S10, training dataset(s) on value(s) of specific target material property(s) of an assessment material are generated for training a machine learning algorithm (MLA). Possible sequence implementations will be described with reference to figures 3a and 3b. There, the generation of training dataset(s) and optional validation dataset(s) and test dataset(s) is described in more detail. In a further step S20, the MLA is trained using the generated training dataset(s). Optionally, the MLA's training halt is determined using the validation dataset(s) and the overall performance is evaluated using the test dataset(s). Possible sequence implementations will be described with reference to figures 4c to 4f. In a further step S30, value(s) of the specific target material property(s) of the assessment material is/are determined using an MLA and training dataset(s). Possible sequence implementations will be described with reference to figures 5a and 5b.

**Figure 3a** shows a flow diagram illustrating an exemplary sequence of substeps for a method for generating material property(s) training dataset(s), corresponding to step S10 in Figure 2.

In a first substep S11 of step S10, a database comprising data records indicative of values of material properties of a multitude of structural materials is provided to the system 1. The structural materials can be steel, aluminum, other nonferrous metals and polymers and other non-metallic materials such as ceramics as well as combinations and compositions of these materials. The material properties can be intrinsic properties as any physical, mechanical, electromagnetic, optical and/or other properties of structural materials. The material properties might also be extrinsic material properties such as material conditions that can include how the material was produced and/or processed (e.g. heat treatment, shape, thickness such as a combination of thermic-, mechanical- and other processing patterns), how one or more of the material properties has/have been measured, how it is used (e.g. temperature range, pressure, humidity) and environmental conditions of use (e.g. corrosive environment, axis of orientation). Another type of material property might also be a material indicator as a material type as defined by a composition (e.g. steel, aluminum etc.), to a higher or lower granularity (e.g. low carbon steel high alloyed, low carbon steel low alloyed etc), and/or by a pre-defined classification (e.g. classification into metals, polymers, ceramics, and composites). The database is either stored in the data storage device 40 or input to the system 1 via the input interface 20. The larger the size and distribution of the dataset(s), and the cleaner the data records, the more accurate a MLA will perform with the processed database.

In a further substep S12, the data records are classified into a plurality of material classes according to the value(s) of one or more of the material properties. There are numerous ways to realize this classification. In particular, a hierarchical taxonomy is set up. For example, a level zero distinguishes metals and non-metals, level 1 distinguishes high level composition differences such as different metal elements and steel, and level 2 distinguishes isotope distribution such as composition of steel. Such a classification can also be performed for different material properties being material condition(s) and combination of material property conditions. Where emphasis should be put and to where a higher granularity of classification is needed for specific materials as well as which combinations of conditions and material properties are to be emphasized is/are determined according to the specific use case(s).

In a further substep S13, target-relevant material properties from the data records corresponding to the specific target material property(s) are extracted within one or more of the material classes. The target-relevant material properties are extracted from the data records corresponding to the specific target material property(s). In the context of the present application, target-relevant material properties are a subset of all material properties in the data records within a material class. A material property in the data records within a material class is part of the subset referred to as target-relevant material properties, if it has any dependency relationship with the specific target material property(s). Hence, the same database, the same material class may be result in a different set of target-relevant material properties for different target material properties. For example, the material property "color" would not be part of the subset of target-relevant material properties - within the material class of metals - for the specific target material property of e.g "tensile strength". However, the same material property "color" would very well be part of the subset of target-relevant material properties - within the material class of metals - for the specific target material property of e.g. "light absorption".

In particular, the extraction of target-relevant material property(s), thus material property(s) relevant for the target material property(s) of the assessment material, can be done by using appropriate algorithms, for instance by applying a mutual information based algorithm, in particular a partial mutual information algorithm to identify a subset of target-relevant material properties of the data records having a dependency relationship with the target material property(s). Alternatively, or additionally, a Markov blanket algorithm is applied onto the data records within one or more of the material classes to identify a subset of target-relevant material properties (out of all material property(s)) of the data records having a dependency relationship with the target material property(s). In particular, applying a Markov blanket algorithm onto the data records within one or more of the material classes comprises identifying a Markov blanket, i.e. the subset of material properties that comprises all data useful for determining the specific target material property(s). In a further substep S14a, the training dataset(s) corresponding to one or more of the material classes are determined. To do so, the values of the extracted target-relevant material properties and corresponding value(s) of the specific target material property(s) are either directly transferred into the training dataset(s) or first subject to normalization before inserted into the training dataset(s). For implementations of normalizing data, see the embodiment below.

At this point, the generated training dataset(s) might be used to train a MLA in the next step S20 depicted in Figure 2, described in more detail with reference to figures 4c to 4f.

Figure 3b shows a further sequence of substeps for a method for generating material property(s) training dataset(s), corresponding to step S10 in Figure 2. Therein, the last substep S14a, now substep S14b comprises not only determining training dataset(s), but also determining validation dataset(s) and/or test dataset(s) corresponding to one or more of the material classes using values of the extracted target-relevant material properties and corresponding value(s) of the specific target material property(s). In particular, as with the determination of training dataset(s), the determination of validation dataset(s) and test dataset(s) may comprise transfer of the data records indicative of values of material properties, or the data records can be subject to normalization before inserted into the training dataset(s). For implementations of normalizing data, see the embodiment below.

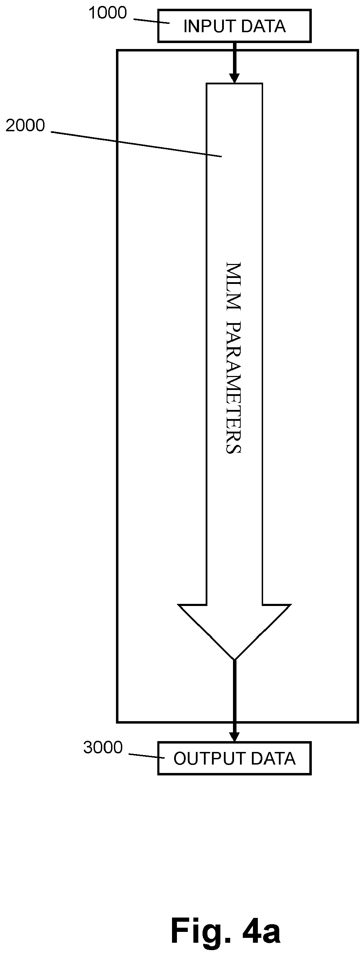

The splitting into the different types of dataset(s) can be performed in different ways. The split may be random in order not to introduce any bias. The split may alternatively be performed using a rational split algorithm, such as selection of data records within one or more of the material classes having a uniform distribution of values of one or more of the material properties over a regression space. In particular, a Kennard-Stone algorithm or other rational split algorithms can be used to produce a training subset, while the remaining data are further randomly split into validation and test datasets. This split is done in order to later be able to train a MLA with the training dataset(s) (as described related to Figures 4a to 4f), determining the halt of the MLA training process with the validation dataset(s) and/or evaluate the trained MLA using the test dataset(s) to predict the underlying assessment material within an accuracy threshold. The split ratio can for instance be as follows: training (70%), validation (20%) and test (10%) dataset(s).

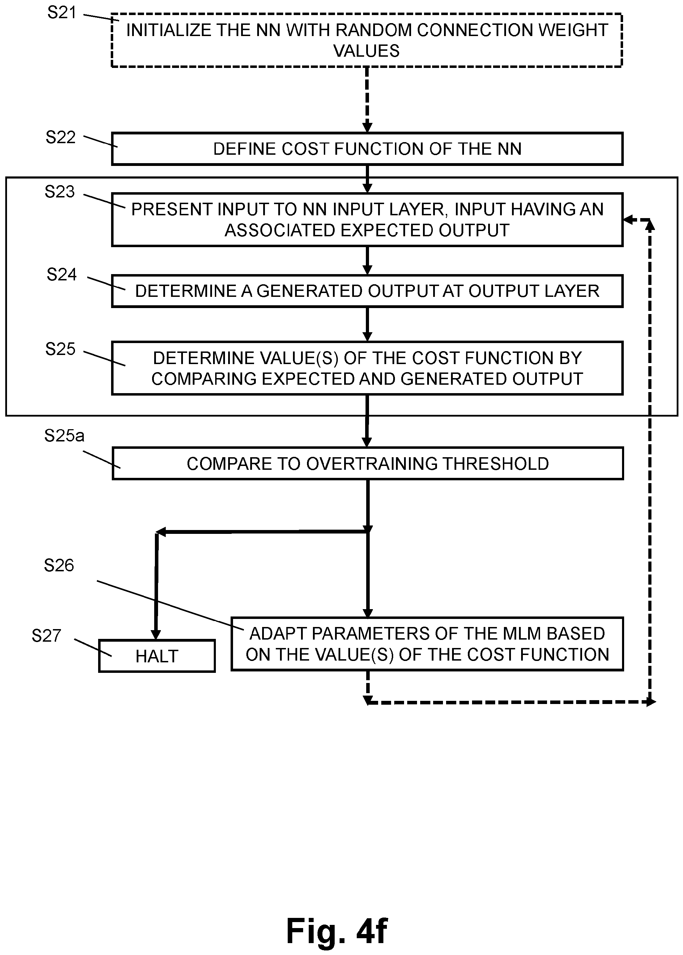

At this point, the generated training, validation and test dataset(s) might be used to train a MLA in the next step S20 depicted in figure 2, described in more detail with reference to figures 4c and 4f.

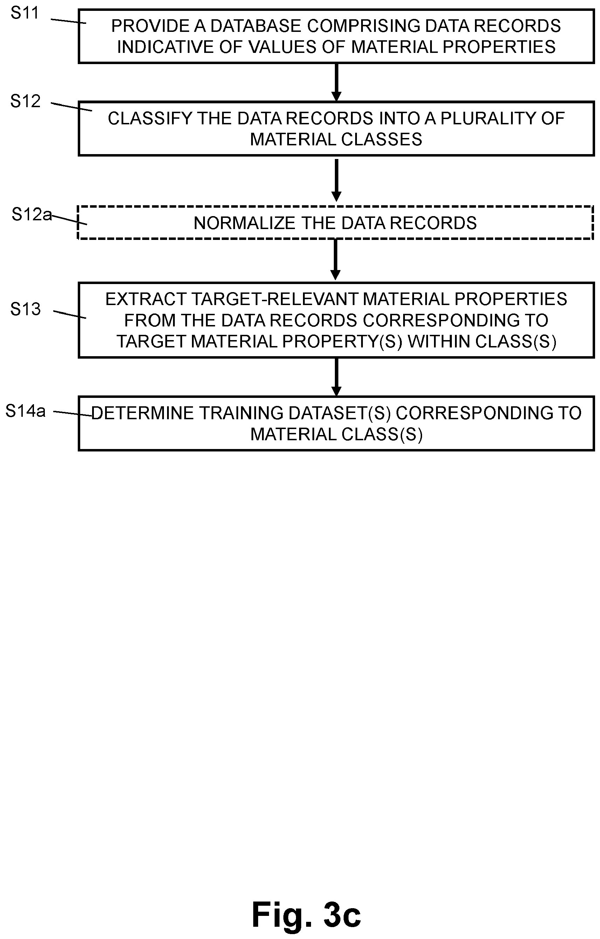

**Figure 3c** shows another flow diagram illustrating an exemplary sequence of substeps for a method for generating material property(s) training dataset(s) based on Figure 3a, wherein in a substep S12a the data records indicative of values of material properties within one or more of the material classes are normalized. In an embodiment, normalizing data records indicative of values of material properties within one or more of the material classes comprises at least one of: removal of data records with missing values of material properties, removal of data records comprising extreme values of material properties, removal of data records comprising inconsistent values of material properties, and transforming categorical values of material properties into corresponding numerical representations. In particular, extreme values are outliers and differ significantly from the majority of other data. Inconsistent values can occur occasionally for some physical and mechanical properties that have substantially different values for same or similar conditions. This can be a consequence of experimental errors, errors in data and unit conversions and the like. The step of transforming non-numerical values of material properties into corresponding numerical representations can be done for example by applying a suitable encoding, such as a binary, numeric or target based encoding. This step may also involve augmentations and high cardinality categorical variables transformation to its numerical representation.

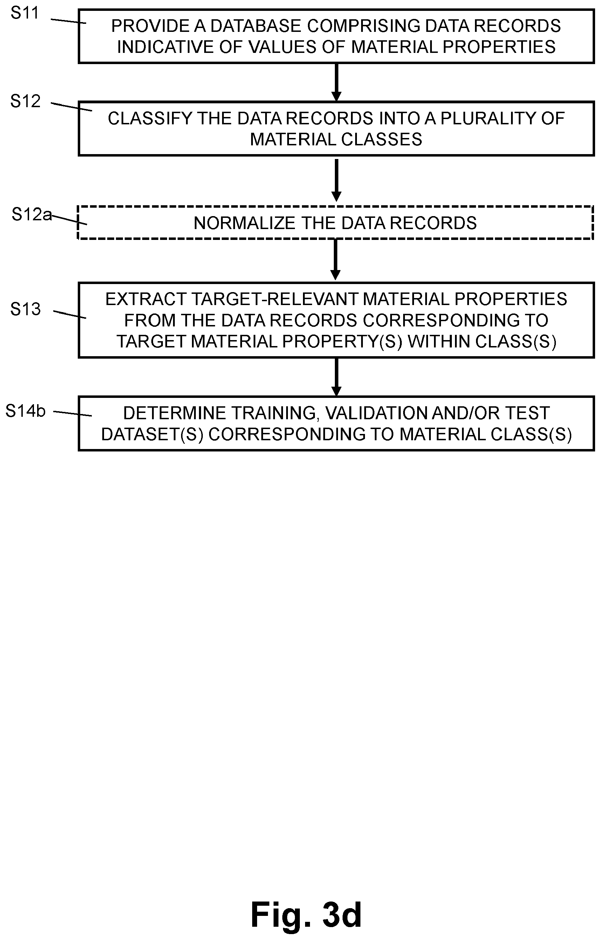

**Figure 3d** shows another flow diagram illustrating an exemplary sequence of substeps for a method for generating material property(s) training dataset(s) based on Figure 3b, wherein in a substep S12a the data records indicative of values of material properties within one or more of the material classes are normalized. Reference is made to the description to Figure 3c above, with the difference on being based on Figure 3b instead of Figure 3a.

**Figure 4a** shows a block diagram illustrating an exemplary, schematic generic machine learning model (MLM) architecture for an exemplary machine learning algorithm (MLA) that is based on such a MLM architecture. A MLM is an architecture (also called a structure) that takes in an external input data 1000, operates on this input with architecture specific parameters 2000 in order to generate output data 3000. Thereby, the parameters 2000 can operate either on the input data 1000 itself or on data that was generated by operating on input data 1000. Furthermore, the architecture can also comprise parameters that are meta parameters. Meta parameters are parameters that do not operate on the input data 1000 itself but define the structure of an implementation of an MLM as well as the form or structure of other parameters. Note that the input data 1000 and the output data 3000 themselves are not part of the MLM and just added to the figure for clarity.

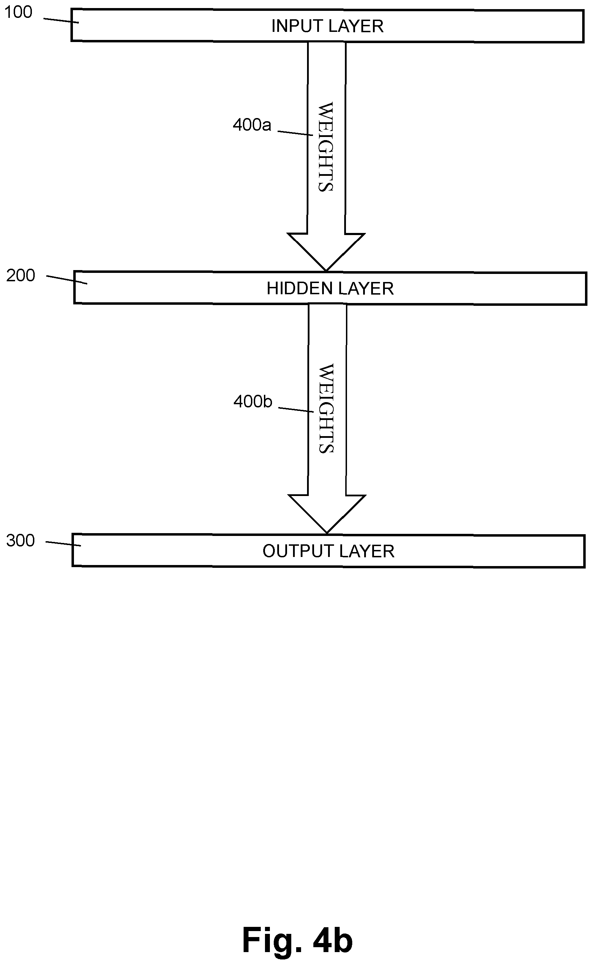

**Figure 4b** shows a block diagram illustrating an exemplary schematic neural network (NN) for a NN-based machine learning algorithm (MLA). A NN is an example of a MLM and input data is operated on by parameters 2000 being connection weights 400a, 400b between layers of the NN. Examples of parameters of a NN are the number of layers, the number of nodes for each of the layers and the connection weights between layers. In the example NN of Figure 4b, the NN comprises an input layer 100, at least one hidden layer 200 and an output layer 300. Usually, the number of successive hidden layers 200 is larger than one. Between any successive layers, there are weight matrices 400a, 400b indicative of the connection weights between successive pairs of layers. The number of hidden layers depends on the NN-based MLA and the underlying dataset(s) and that the number of hidden layers can also be an optimizable meta parameter of the NN-based MLA.

**Figure 4c** shows a flow diagram illustrating an exemplary sequence of substeps for a method of training a machine learning algorithm (MLA) for determining material properties of an assessment material using training dataset(s). To do so, a regression model representative of a relationship between values of two or more material properties is computed. In a most basic case, parameters and a cost function are predefined.

In a substep S201, a dataset of input and expected output pairs (xi, yiexp) is presented to the MLM architecture. In a further substep S202, generated output yi after having run through the MLM architecture is determined. By reaching the output, the data has been processed by all of the parameters. In a substep S203, the output yi generated in substep S202 is compared to the corresponding expected output yiexp by determining value(s) of the cost function.

In a substep S204, the MLM architecture parameters are adapted based on the value(s) of the cost function. The adaption of the parameters is intended to minimize the cost function. In order to achieve this, there are different methods that are specific for the underlying MLM architecture.

Optionally, and typically, the previous substeps are repeated with the adapted MLM architecture parameters.

After having achieved a minimized cost function below a certain threshold, and optionally after the MLM has been tested with the test dataset(s) below a certain threshold, the MLM architecture can be coded and compiled, referred to as a "trained" machine learning algorithm or a machine learning model.

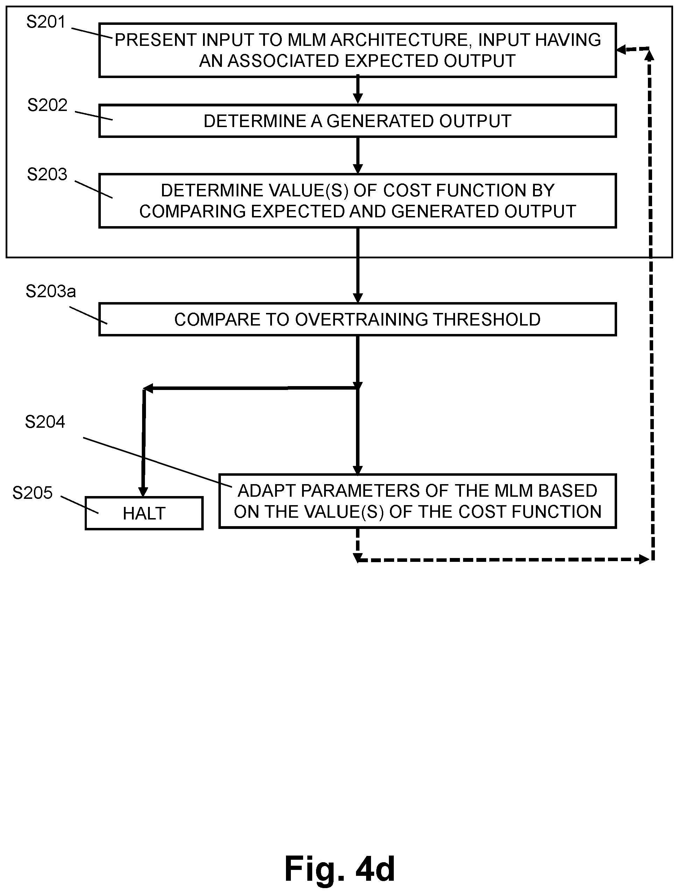

**Figure 4d** shows another flow diagram illustrating an exemplary sequence of substeps for a method of training a machine learning algorithm (MLA) for determining material properties of an assessment material using training dataset(s). The steps S201 to S203 are performed as described above, this time for both the training dataset(s) and the validation dataset(s). In a further substep S203a, the determined values of the cost function are compared to an overtraining threshold. In case of not having reached the threshold yet, the parameters are adapted in S204. This adaption is done based on the value(s) of the cost function of the training dataset(s) as described before. As described above, the steps S201 to S203 are repeated with the adapted parameters. In repeating these steps, intermediary sets of parameters and cost function value(s) are kept and saved. In case of reaching the overtraining threshold, the MLA comes to a halt in step S205. The overtraining threshold can be implemented for instance by a direct comparison of the values at which the error of the validation dataset exceeds the value of the training dataset(s). The overtraining threshold can also be implemented as a threshold of a rising error of the validation dataset(s). The overtraining threshold can also be implemented by a combination of the two previous examples.

After having achieved a minimized cost function below a certain threshold and after halting the training process at an overtraining threshold, the MLM architecture can be coded and compiled, referred to as a "trained" machine learning model.

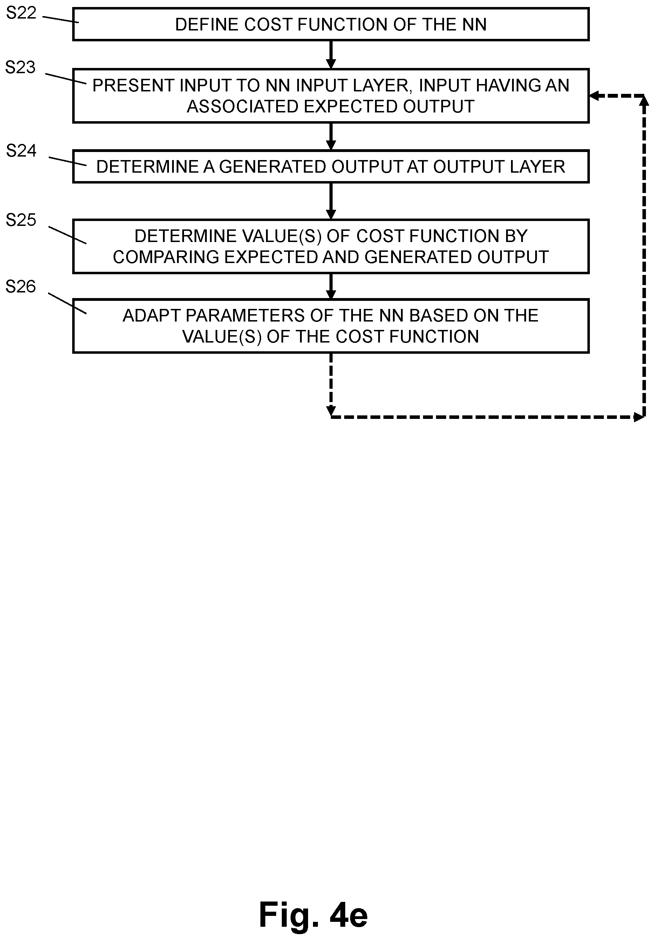

**Figure 4e** shows a flow diagram illustrating an exemplary sequence of substeps for a method of training a neural network (NN)-based machine learning algorithm (MLA) for determining material properties of an assessment material using training dataset(s).

In a substep S22, a cost function of the NN is defined. The cost function is an important parameter to determine the accuracy of a MLA for given dataset(s) and that the goal of training a NN is to minimize the cost function. Different functions can be used, typically absolute or relative mean error, mean absolute percentage error (MAPE), mean squared error (MSE) or multi-class classification cost functions or a combination thereof are used.

In a substep S23 a dataset of input and expected output pairs (xi, yiexp) is selected, the input being presented to the input layer 100 of the NN. Therein, the input xi comprises a first subset of material properties of any one of the plurality of data records of the training dataset(s) and the expected output yiexp comprises a second subset of material properties of the plurality of data records of the training dataset(s).

In a substep S24, a generated output yi at the output layer 300 of the NN after having run through the NN is determined. The input xi is thereby processed by the NN in that the current data within a layer 100, 200, ... is processed by the weight matrices 400a, 400b, ... by stepping from one layer to the successive layer. By reaching the output layer 300, the data has been processed by all of the weight matrices 400a, 400b, ... of the NN.

In a substep S25, the output yi generated in substep S24 is compared to the corresponding expected output yiexp by determining value(s) of the cost function.

In a substep S26, the NN is adapted based on the value(s) of the cost function. The adaption can include updating weights and/or biases of the NN. The adaption of the parameters is intended to minimize the cost function. In order to achieve this, there are different methods depending on the neural network architecture.

Optionally, and typically, the previous substeps are repeated with the adapted NN parameters.

**Figure 4f** shows another flow diagram illustrating an exemplary sequence of steps for a method of training a machine learning algorithm for determining material properties of an assessment material using training dataset(s). In addition to the method presented in Figure 4e, in a preparatory step S21, the NN is initialized with random connection weight values in the weight matrices. Figure 4f further shows all the steps performed in figure 4e. This time, steps S23 to S25 are performed for the training and the validation dataset(s).

In a further substep S25a, the determined values of the cost function are compared to an overtraining threshold to halt the training process before doing overtraining as described in figure 4e. In case of not having reached the threshold yet, the parameters of the MLM are adapted based on the value(s) of the cost function of the training dataset(s).

**Figure 5a** shows a flow diagram illustrating an exemplary sequence of substeps for a method for determining target material property(s) of an assessment material using a machine learning algorithm (MLA).

In a substep S31, input indicative of a first set of material properties of an assessment material is received, in particular via the input interface 20. This set of material properties comprises material property(s) to which the following inquiry shall be constrained to as well as material property(s) that shall be inquired in substep S32.

In a further substep S32, a second set of material properties (different from the first set) is determined. In this set are the value(s) of the specific target material property(s). They are determined using a MLM of the MLA trained according to any one of the embodiments of the method for training a MLA, using training dataset(s) generated according to any one of the embodiments of generating training dataset(s).

In particular, a possible implementation of the above two steps might be as described in the following. The first (input) set of material properties might comprise material condition(s) and a material type or composition as input material properties and a mechanical property as specific target material property(s). A MLA is chosen for determination of the value of the specific target material property(s), the chosen MLA being the one that was trained with training dataset(s) generated with respect to material class(s) classified according to these constraining material property(s) as described in the previous figures 3-4. The constraining part of the set of material property(s) is then run through the chosen MLA to determine the target material property(s), in this example a mechanical property of the assessment material. To give a more specific example, the input material property can comprise a type of material, heat treatment with its parameters and product for metals. A set of corresponding material properties of the training dataset(s) then comprises range(s) equal to and/or comprised by and/or overlapping with range of the condition(s) and type of metal. Thereafter, the value(s) of the specific target material property(s) of the assessment material is determined using the chosen MLA.

**Figure 5b** shows another flow diagram illustrating an exemplary sequence of substeps for a method for determining target material property(s) of an assessment material using a machine learning algorithm (MLA).

In an optional substep S31a, a selection of a machine learning algorithm is received. The selection can either be done by the application by choosing an MLA with an MLM or combination of MLMs that had the best performance according to a predefined performance function. Or the selection can be selected by a user and therefore input via the input interface 20. Therein, the MLA is generated and trained in accordance with the selection of a MLM. The selection of a MLM or a combination of MLMs amongst a plurality of MLMs defines structure and the nature of the parameters of the MLA. The second set of material properties is then determined using the MLA according to the selected MLM or MLM combination.

In further optional substep S33, a data output is generated, indicative of the value(s) of the specific target material property(s) of the assessment material. Thereafter, in another optional substep S34, a visual representation of at least part of the data output is displayed on a display device. Alternatively, or additionally, a product is manufactured comprising the assessment material on the basis of knowledge of the value(s) of the specific target material property(s) for the assessment material.

It should be noted that, in the description, the computer program code has been associated with specific functional modules and the sequence of the steps has been presented in a specific order, one skilled in the art will understand, however, that the computer program code may be structured differently and that the order of at least some of the steps could be altered, without deviating from the scope of the disclosure.

**REFERENCE LIST**

| | |
|---|---|
| system (for processing material properties of structural materials) | 1 |
| computing device | 10 |
| input interface | 20 |
| output interface | 30 |
| data storage device | 40 |
| display device | 50 |
| input layer (of neural network) | 100 |
| hidden layer (of neural network) | 200 |
| output layer (of neural network) | 300 |
| connection weights (between layers of neural network) | 400a, 400b |
| input data | 1000 |
| architecture specific parameters | 2000 |
| output data | 4000 |

## Claims

1. A computer implemented method for processing material properties of structural materials, the method comprising generating training dataset(s) for training a machine learning algorithm for determining value(s) of specific target material property(s) of an assessment material, the generation of training dataset(s) comprising the steps:
- providing a database comprising data records indicative of values of material properties of a multitude of structural materials;
- classifying the data records into a plurality of material classes according to the value(s) of one or more of the material properties;
- within one or more of the material classes, extracting target-relevant material properties from the data records corresponding to the specific target material property(s);
- determining the training dataset(s) corresponding to one or more of the material classes using values of the extracted target-relevant material properties and corresponding value(s) of the specific target material property(s).

2. The computer implemented method for processing material properties of structural materials according to claim 1, further comprising determining validation and/or test dataset(s) corresponding to one or more of the material classes using values of the extracted target-relevant material properties and corresponding value(s) of the specific target material property(s).

3. The computer implemented method for processing material properties of structural materials according to claim 2, wherein splitting one or more of the material classes into training, validation and/or test dataset(s) is performed using a rational split algorithm, such as selection of data records within one or more of the material classes having a uniform distribution of values of one or more of the material properties over a regression space.

4. The computer implemented method for processing material properties of structural materials according to one of the claims 1 to 3, wherein extracting target-relevant material properties from the data records indicative of material properties of one or more of the material classes comprises:
- applying a mutual information based algorithm, in particular a partial mutual information algorithm and/or
- applying a selection algorithm such as a Markov blanket algorithm
onto the data records within one or more of the material classes to identify a subset of target-relevant material properties of the data records having a dependency relationship with the target material property(s).

5. The computer implemented method for processing material properties of structural materials according to claim 1 or 4, wherein generating training dataset(s) further comprises normalizing the data records within one or more of the material classes, wherein normalizing the data records within one or more of the material classes comprises:
- removal of data records with missing values of material properties; and/or
- removal of data records comprising extreme values of material properties; and/or
- removal of data records comprising inconsistent values of material properties; and/or
- transforming non-numerical values of material properties into corresponding numerical representations.

6. The computer implemented method for processing material properties of structural materials according to one of the claims 1 to 5, further comprising determining respective applicability domain(s) of one or more of the training dataset(s).

7. The computer implemented method for processing material properties of structural materials according to one of the claims 1 to 6, further comprising training of a machine learning algorithm for determining the specific target material property(s) of an assessment material using the training dataset(s).

8. The computer implemented method for processing material properties of structural materials according to claim 7, wherein the training of the machine learning algorithm comprises computing a regression model representative of a relationship between values of two or more of material properties within the training dataset(s).

9. The computer implemented method for processing material properties of structural materials according to claim 7 or 8,
wherein the machine learning algorithm comprises a neural network-based machine learning algorithm, and
wherein the training of the machine learning algorithm further comprises the steps of:
a) receiving a first set of material properties as an input, a second set of material properties as an expected output associated with the input;
b) determining a generated output of the neural network by inputting the input into the neural network;
c) determining value(s) of a selected cost function based on a comparison of the expected output and the generated output; and
d) adapting the neural network based on the value(s) of the selected cost function

10. The computer implemented method for processing material properties of structural materials according to claim 8 or 9, wherein the machine learning algorithm comprises a decision tree-based machine learning algorithm, in particular a gradient-boosted decision tree-based machine learning algorithm.

11. The computer implemented method for processing material properties of structural materials according to one of the claims 7 to 10, further comprising preventing overtraining of the machine learning algorithm using the validation dataset(s) and/or further comprising evaluating the performance of the machine learning algorithm using the test dataset(s).

12. The computer implemented method for processing material properties of structural materials according to one of the claims 1 to 11, further comprising determining value(s) of the specific target material property(s) of an assessment material using the machine learning algorithm.

13. The computer implemented method for processing material properties of structural materials according to claim 12, wherein determining value(s) of the specific target material property(s) using the machine learning algorithm comprises the steps:
- receiving input indicative of value(s) of a first set of material properties of the assessment material; and
- determining value(s) of the specific target material property(s) of the assessment material using the machine learning algorithm trained using the training dataset(s).

14. The computer implemented method for processing material properties of structural materials according to claim 13, wherein determining value(s) of target material property(s) using the machine learning algorithm further comprises selecting a machine learning algorithm trained with a training dataset for the specific target material property(s).

15. The computer implemented method for processing material properties of structural materials according to claim 13 or 14, wherein determining value(s) of target material property(s) using the machine learning algorithm further comprises receiving a selection of a machine learning model, wherein:
- the machine learning algorithm is generated and trained in accordance with the selection of a machine learning model; and
- the values of specific target material property(s) of the assessment material is/are determined using the machine learning algorithm according to the selected machine learning model.

16. The computer implemented method for processing material properties of structural materials according to one of the claims 12 to 15, further comprising generating a data output indicative of the values of the target material property(s) of the assessment material.

17. The computer implemented method for processing material properties of structural materials according to claim 16, further comprising displaying a visual representation of at least part of the data output on a display device (50) and/or manufacture of a product comprising the target material property in accordance with the determined values of the target material property(s) of the assessment material.

18. A system (1) for processing material properties of structural materials comprising:
- a data storage device (40) for storing a database comprising data records indicative of values of material properties of a multitude of structural materials, and for storing training dataset(s);
- a computing device (10) communicatively connected to the data storage device;
- an input interface (20) for receiving input indicative of value(s) of a first set of material properties of the assessment material and/or for receiving a selection of a machine learning model; and
- an output interface (30) for outputting output data indicative of values of target material property(s) of an assessment material,
wherein the system (1) is configured to carry out the method for processing material properties of structural materials according to one of the claims 1 to 17.

19. The system (1) for processing material properties of structural materials according to claim 18, further comprising a display device (50) communicatively coupled to the output interface (30) for displaying a visual representation of the output data.

20. A computer program product comprising instructions which, when executed by a computing device (10), cause the computing device (10) to carry out the method according to any one of the claims 1 to 17.
